(19) **Europäisches Patentamt / European Patent Office / Office européen des brevets**

(11) **EP 3 327 427 A1**

(12) **EUROPEAN PATENT APPLICATION**
published in accordance with Art. 153(4) EPC

(43) Date of publication:
**30.05.2018 Bulletin 2018/22**

(21) Application number: **16827756.4**

(22) Date of filing: **15.07.2016**

(51) Int Cl.:
**G01N 21/64** (2006.01)     **G01N 33/48** (2006.01)
**G01N 33/53** (2006.01)     **G01N 33/533** (2006.01)
**G02B 21/00** (2006.01)

(86) International application number:
**PCT/JP2016/071037**

(87) International publication number:
**WO 2017/014196 (26.01.2017 Gazette 2017/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **17.07.2015 JP 2015142813**

(71) Applicant: **Konica Minolta, Inc.**
**Tokyo 100-7015 (JP)**

(72) Inventors:
• **TOMIOKA, Daisuke**
**Tokyo 100-7015 (JP)**

• **GODA, Hideki**
**Tokyo 100-7015 (JP)**
• **SATO, Akira**
**Tokyo 100-7015 (JP)**
• **OZAKI, Yuichi**
**Tokyo 100-7015 (JP)**
• **TAKAHASHI, Masaru**
**Tokyo 100-7015 (JP)**

(74) Representative: **Hoffmann Eitle**
**Patent- und Rechtsanwälte PartmbB**
**Arabellastraße 30**
**81925 München (DE)**

(54) **TARGET BIOLOGICAL SUBSTANCE ANALYSIS METHOD AND ANALYSIS SYSTEM**

(57) The present invention addresses the problem of providing a target biological substance analysis system, whereby a number of fluorescent bright points for each immunostaining agent is accurately analyzed. An analysis system 1 for the target biological substance is a system for staining a tissue specimen 30 with a plurality of types of immunostaining agents including mutually different fluorescent nanoparticles and analyzing the tissue specimen 30 using a fluorescence microscope 10. In the analysis system 1, there are provided a fluorescence microscope 10 for capturing an image of the tissue specimen 30 stained by the immunostaining agent, and a control device 60 for controlling the fluorescence microscope 10. The control device 60 has a storage unit 64 for storing a luminance ratio for each filter set 50 of each of the immunostaining agents, and a control unit 62 for measuring a number of fluorescent bright points of the tissue specimen 30 for each filter set 50 from the results of image capture by the fluorescence microscope 10, and computing the number of fluorescent bright points for each immunostaining agent on the basis of the luminance ratio thereof.

FIG.1

**Description**

**Technological Field**

The present invention relates to an analysis method and an analysis system of a target biological substance.

**Description of the Related Art**

According to a conventional immunostaining of a tissue slice, a fluorescent substance modified with an antibody (immunostaining reagent) is bonded to an antigen to be observed for labelling and observing with a fluorescence microscope.

In a pathological examination relative to cancer by immunostaining, diagnosis can be performed as for poor prognosis after anticancer treatments etc. by examining whether each of the multiple kinds of antigens on one tissue slice is positive or negative.

Regarding such pathological examination, the applicant of the present invention has been providing detection methods of a biological substance and immunostaining reagents which enable measurement of expression level of antigen based on the analysis result of the number of fluorescent bright points and fluorescence intensity for each antigen (see EXAMPLE 3 in Patent Document 1).

**Prior Art Document**

**Patent Document**

Patent Document 1: International Publication No. 2012/029342

**Summary**

**Problems to be Solved by the Invention**

In a fluorescent microscopic observation, a filter set(s) corresponding to the kind of the immunostaining reagent (excitation wavelength and emission wavelength of the fluorescent substance) is placed at a predetermined position of lens barrel.

However, in the case that multiple kinds of immunostaining reagents are used in the fluorescent microscopic observation as described above, fluorescence from multiple kinds of immunostaining reagents are observed in the fluorescent microscopic observation using one filter set. Therefore, it has become apparent that accurate analysis (calculation) is not possible as for the number of fluorescent bright points for each immunostaining reagent or as for the number of fluorescent substance particles calculated based on the fluorescent bright points.

Accordingly, the main object of the present invention is to provide an analysis method and an analysis system of a target biological substance which enable accurate analysis of the number of fluorescent bright points for each immunostaining reagent even in multiple-immunostaining using multiple kinds of immunostaining reagents.

**Means for Solving the Problem**

In order to solve the above-mentioned problems, according to the first embodiment of the present invention, there is provided an analysis method of a target biological substance for analyzing a tissue specimen with a fluorescence microscope, the tissue specimen being stained with immunostaining reagents including different kinds of fluorescent nanoparticles, the analysis method including:

calculating a luminance ratio of each of the immunostaining reagents for each of filter sets; and
measuring number of fluorescent bright points in the tissue specimen for each of the filter sets for calculating number of fluorescent bright points for each of the immunostaining reagents based on the luminance ratio.

According to another embodiment of the present invention, there is provided an analysis system of a target biological substance for analyzing a tissue specimen with a fluorescence microscope, the tissue specimen being stained with immunostaining reagents including different kinds of fluorescent nanoparticles, the system including:

a fluorescence microscope to image the tissue specimen stained with the immunostaining reagents,
a controller controlling the fluorescence microscope and including:

a storage to store luminance ratio of each of the immunostaining reagents for each of filter sets; and
a calculator to measure number of fluorescent bright points in the tissue specimen for each of the filter sets and to calculate number of fluorescent bright points for each of the immunostaining reagents based on the luminance ratio.

## Advantageous Effects of Invention

According to the present invention, it is possible to accurately analyze the number of fluorescent bright points for each immunostaining reagent.

## Brief Description of the Drawings

FIG. 1 is a diagram illustrating a schematic configuration of an analysis system for an objective biological substance;
FIG. 2 is a flowchart sequentially showing an analysis method of a target biological substance;
FIG. 3 is a diagram schematically describing calculation step of luminance ratios;
FIG. 4 is a graph illustrating an exemplary calculation method of a luminance ratio;
FIG. 5 is a diagram schematically describing calculation step of numbers of fluorescent bright points;
FIG. 6 is a diagram schematically illustrating relation of an excitation filter and a fluorescence filter in a filter set;
FIG. 7 is a diagram schematically illustrating relation of fluorescence filters in filter sets; and
FIG. 8 is a diagram schematically describing overlapping wavelength of emission spectra of fluorescent nanoparticles.

## Detailed Description Of Embodiments

Hereinafter, preferred embodiments of the present invention will be explained with reference to the drawings.
As used herein, the term "to" between two numerical values indicates that the numeric values before and after the term are inclusive as the lower limit value and the upper limit value, respectively.

[ANALYSIS SYSTEM OF TARGET BIOLOGICAL SUBSTANCE]

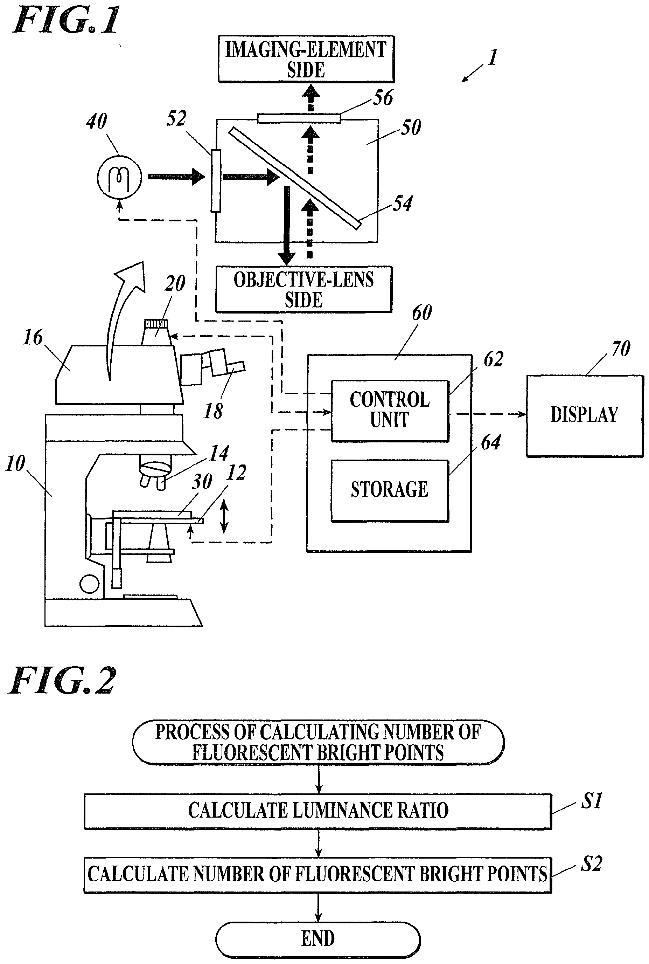

As illustrated in FIG. 1, an analysis system 1 of a target biological substance(s) is provided with a fluorescence microscope 10, a controller 60, and a display 70.
The fluorescence microscope 10 is equipped with a stage 12, an objective lens 14, a lens barrel 16, an ocular lens 18, and an imaging element 20. Tissue specimen 30 subjected to immunostaining is put on the stage 12. The lens barrel 16 incorporates a lamp 40 and a filter set 50. The filter set 50 includes an excitation filter 52, a beam splitter 54, and a fluorescence filter 56.
The lamp 40 emits excitation light. The excitation filter 52 transmits the excitation light only. The beam splitter 54 is an optical component for reflecting or transmitting light depending on its wavelength, using a predetermined wavelength as a border. In the present embodiments, the beam splitter 54 reflects the excitation light and transmits the fluorescent light. The fluorescence filter 56 blocks the excitation light and transmits the florescent light only.
In the fluorescence microscope 10, when the lamp 40 is turned on, the excitation light passes through the excitation filter 52, is reflected by the beam splitter 54, and passes through the objective lens 14, so that the tissue specimen 30 is irradiated with the excitation light. The resulting fluorescence emitted from the tissue specimen 30 is condensed by the objective lens 14 and passes through the beam splitter 54 and the fluorescence filter 56. Subsequently, the fluorescence is observed through the ocular lens 18 as a fluorescent image and imaged by the imaging element 20.
The fluorescence microscope 10 is connected to the controller 60 controlling them.
The controller 60 is equipped with a control unit 62 and a storage 64.
The control unit 62 is connected to the stage 12 and can control the focal position (height position) by controlling the lifting and lowering of the stage 12. The control unit 62 is connected to the imaging element 20 and can generate a fluorescent picture from a fluorescent image by controlling the imaging element 20 to image the fluorescent image. The control unit 62 is connected to the lamp 40 and can control the turning on and off of the lamp 40. The storage 64 stores a program for analyzing target biological substance to carry out the calculation process of the number of fluorescent bright points in FIG. 2.
The controller 60 is connected to the display 70 which displays the calculation results by the controller 60 and the like.

[TISSUE SPECIMEN]

Next, the tissue specimen 30 will be explained.

The tissue specimen 30 is a tissue slice including the target biological substance(s) and stained with an immunostaining reagent(s). The stained tissue specimen 30 is put on the stage 12 of the fluorescence microscope 10.

(1) TARGET BIOLOGICAL SUBSTANCE

The target biological substance(s) is a biological substance expressing on a tissue slice, especially protein (antigen), and is subjected to immunostaining using a fluorescent marker(s) for detection or quantitation mainly in view of pathological diagnosis.

A typically used target biological substance is expressed on cell membrane of various cancer tissues and can be used as a biomarker.

(2) IMMUNOSTAINING REAGENT (ANTIGEN-FLUORESCENT NANOAPARTICLE CONJUGATE)

For improving the efficiency of fluorescence labelling and reducing the time which results in deterioration of fluorescence, the immunostaining reagent is preferably a complex in which a primary antibody is connected to a fluorescent nanoparticle indirectly, that is, not by a covalent bond but by a bond using antigen-antibody reaction and the like. In order to make staining operation easier, the immunostaining reagent may be a complex in which a primary antibody or a secondary antibody is connected to a fluorescent nanoparticle directly.

An exemplary immunostaining reagent may be described as follows: [a primary antibody against the objective biological substance] ... [an antibody (secondary antibody) against the primary antibody] ~ [a fluorescent nanoparticle (hereinafter, also referred to as a "resin particle including fluorescent dye")]

"..." represents a bond by an antibody-antigen interaction. "~" represents a non-limited bond, for example, a covalent bond, an ionic bond, a hydrogen bond, a coordination bond, an antibody-antigen interaction, a biotin-avidin interaction, physical adsorption, chemical adsorption, and the like. If necessary, a bond via a linker molecule may be used.

Other than the embodiments in which a primary antibody is connected to a fluorescent nanoparticle indirectly, that is, other than the bond by antigen-antibody reaction, the embodiment of the immunostaining reagent for target biological substance includes [a primary antibody labeled with hapten] ... [an antibody against hapten (anti-hapten antibody)] ~ [fluorescent nanoparticle] using hapten molecule having high bonding ability and [a primary antibody labeled with biotin] ... [avidin] ~ [fluorescent nanoparticle] also having high bonding ability. Specifically, the compounds regarding the indirect reaction include hapten, anti-hapten antibody (dinitrophenol, anti-dinitrophenol antibody, digoxigenin, anti-digoxigenin antibody, fluorescein, anti-fluorescein antibody, and the like), biotin, avidin, and modified avidin (Streptavidin, NeutrAvidin, and the like). Otherwise, an embodiment regarding FISH includes forming a complex containing a nucleic acid (prove) bonded to hapten and a resin particle including fluorescent dye bonded to a hapten-affinitive molecule.

In the embodiment of indirect bond using the affinitive molecule, a polymer having functional groups at both ends is used. The functional groups of the polymer respectively react with a functional group of the resin and a functional group of the affinitive molecule. The resin particle including fluorescent dye and the affinitive molecule are thereby bonded to each other via a spacer (linker) derived from the polymer. The present invention is highly effectively applied especially in such embodiment, because of the easy variation in the amount of the affinitive molecule bonding to a unit area of the surface of the resin particle including fluorescent dye. For example, using amidation by reaction of amine and carboxylic acid, sulfidation by reaction of maleimide and thiol, imination by reaction of aldehyde and amine, amination by reaction of epoxy and amine, and the like, a first affinitive molecule (with a spacer as needed) and the resin particle including fluorescent dye can be covalently bonded. Functional groups regarding the above-described reaction are respectively introduced to the first affinitive molecule, the both ends of the spacer used as needed, and the surface of the resin particle including fluorescent dye and react with each other in a predetermined condition to form a connection via a covalent bond. The first affinitive molecule means an affinitive molecule bonded to the surface of the resin particle including fluorescent dye as described above.

As the spacer, hydrophilic polymer is preferable because unspecific adsorption can be reduced and the like. In particular, polyethylene glycol (PEG) is preferable because chain length can be easily controlled by the number of oxyethylene units. A commercially available PEG can be purchased from, for example, Thermo Fisher Scientific Inc., and PEG having an arbitrary length can be obtained by requesting a manufacturer of reagents to manufacture such PEG by specifying the number of repeating units in the chemical structure.

The length of the spacer may be controlled within a suitable range according to the particle size of the resin particle including fluorescent dye to which the spacer is bonded. The spacers may have a single kind of length, but may have two or more kinds of length. When spacers have a single kind of length, it is preferably 1 to 100nm. When spacers have two or more kinds of length, at least one kind of the length of the spacer is preferably 1 to 100 nm and the other kind(s) of the length of the spacer is preferably 0.1 to 10 nm.

A significant factor to determine the quality of immunostaining reaction includes biocompatibility. The biocompatibility can be evaluated as follows. A fixed amount of a solution including a fixed concentration of resin particles including fluorescent dye having a surface bonded with the first affinitive molecule (representatively avidin) is reacted with a plate having a fixed concentration of a molecule (hereinafter, such molecule is also referred to as "a second affinitive molecule" and is exemplified by, but not limited to, biotin corresponding to avidin) which can specifically bond to the first affinitive molecule. When a signal value (S) measured from the solution during the reaction satisfies the following formula, the solution is selected to be used according to the manufacturing and evaluating method of the solution of the resin particles including fluorescent dye.

$$0.5 \times A \times B \times (C/D) \leq S \leq 1.2 \times A \times B \times (C/D)$$

A: The signal value measured during reaction of the plate and the fluorescent dye molecules alone as many as those in all the resin particles including fluorescent dye bonded with the first affinitive molecule and included in the fixed amount of the solution of the fixed concentration.

B: The number of the first affinitive molecules bonded to one resin particle including fluorescent dye, calculated by dividing the total number of the first affinitive molecules bonded to the entire surface of all the resin particles including fluorescent dye included in the fixed amount of the solution of the fixed concentration by the number of the resin particles including fluorescent dye.

C: The emission intensity per one resin particle including fluorescent dye.

D: The emission intensity of the fluorescent dye molecules alone as many as those in one resin particle including fluorescent dye

By such manufacturing and evaluating method, the solution of the resin particles including fluorescent dye having sufficient staining property can be selected. Furthermore, the concentration of the resin particles including fluorescent dye is controlled on the basis of the signal value obtained by a predetermined method. The solution of the resin particles including fluorescent dye having a constant staining property can be thereby obtained. Using biotin as the second affinitive molecule, a biotinylated plate, and resin particles including fluorescent dye after surface modification by SA, for example, the signal value from the plate is highly correlated with the luminance intensity obtained by actual immunostaining using the particles. The staining property of the particles can be thereby determined without actual staining. Furthermore, by diluting the solution so that the ratio(S/T) is a predetermined value (S is the signal measured when the solution of the resin particles including fluorescent dye is dispersed on the biotinylated plate and T is the total surface area of the resin particles including fluorescent dye), it is possible to obtain the solution having a fixed staining property by adjusting the concentration of the solution of the resin particles including fluorescent dye. Accordingly, it is possible to obtain pathological staining results without variations between solutions of the resin particles including fluorescent dye of different lots. The following calculation can provide the 'amount of the fluorescent dye in all the resin particles including fluorescent dye bonded with the first affinitive molecule and included in the fixed amount of the solution of the fixed concentration' (a) of the fluorescent dye described in above definition "A". A fixed amount of a solution including a fixed concentration (for example, 0.01 nM, that is, $6.02 \times 10^9$ particles/mL) of the resin particles including fluorescent dye (a standard solution of resin particles including fluorescent dye) and a solution including the same fixed concentration (for example, 0.01 nM, that is, $6.02 \times 10^9$ particles/mL) of the fluorescent dye (a standard solution of fluorescent dye) are prepared. The fluorescence intensity of each solution is measured at the emission peak wavelength. Because the fluorescent dye is accumulated at a high concentration in the resin particles including fluorescent dye, the fluorescence emitted from the resin particles including fluorescent dye may be reduced by a phenomenon called concentration quenching. By multiplying the "fluorescence intensity of the standard solution of resin particles including fluorescent dye" by "(quantum yield of the fluorescent dye molecules alone)/(quantum yield of the resin particle including fluorescent dye)", the fluorescence intensity of the standard solution of resin particles including fluorescent dye after correcting the influence by the concentration quenching can be calculated. The 'amount of the fluorescent dye in all the resin particles including fluorescent dye bonded with the first affinitive molecule and included in the fixed amount of the solution of the fixed concentration' can be obtained by dividing the "the fluorescence intensity of the standard solution of resin particles including fluorescent dye after correction" by the "fluorescence intensity of the standard solution of fluorescent dye", in other words, by the following equation: {(fluorescence intensity of the standard solution of resin particles including fluorescent dye)/(fluorescence intensity of the standard solution of fluorescent dye)} × {(quantum yield of the fluorescent dye molecules alone)/(quantum yield of the resin particle including fluorescent dye)}. The quantum yield can be measured with a publically-known method, for example, using "Quantaurus-QY" (Absolute PL quantum yield spectrometer, made by Hamamatsu Photonics K.K.)

After calculating the above value (a), a solution including the amount of the fluorescent dye is prepared and applied to a plate on which the second affinitive molecule is fixed for reaction. The resulting fluorescence intensity (signal value) (A) can be thereby measured.

The "total number of the first affinitive molecules bonded to the entire surface of all the resin particles including fluorescent dye included in the fixed amount of the solution of the fixed concentration" (b1) described in above definition "B" can be calculated by dividing the total weight of the first affinitive molecule bonded to the entire surface by the molecular weight of the first affinitive molecule. While the first affinitive molecule represented by Streptavidin is a protein, the base material of the resin particle including fluorescent dye is resin. Accordingly, the concentration of the protein in the fixed amount of the solution of the resin particles including fluorescent dye can be measured based on the quantitation method of protein, such as BCA method (all the protein is assumed to be bonded to the surface of the resin particles including fluorescent dye). By multiplying the measured concentration by the volume of the "fixed amount of the solution", total weight of the protein can be calculated. By dividing the total weight by the molecular weight of the protein (for example, the molecular weight of Streptavidin is 52000), the total number of the protein molecules (the first affinitive molecule) used in the definition "B" can be calculated.

The "number of the resin particles including fluorescent dye" (b2) included in the fixed amount of the solution of the fixed concentration described in the above definition "B" can be measured with a particle counter (for example, "Liquid Particle Counter" manufactured by RION Co., Ltd.). By dividing the total number of the first affinitive molecule in the previous paragraph by the number of the resin particles including fluorescent dye, the number of the first affinitive molecules bonded to one resin particle including fluorescent dye can be calculated.

After calculating the values (b1) and (b2) described above, the number (the averaged value) (B) of the first affinitive molecules bonded to one resin particle including fluorescent dye can be obtained by calculating (b1)/(b2).

The "emission intensity per one resin particle including fluorescent dye" in above definition "C" can be calculated as follows. Using a particle counter, a fixed amount of a solution including a fixed concentration (for example, 0.01 nM, that is, $6.02\times10^9$ particles/mL) of the resin particles including fluorescent dye (a standard solution of resin particles including fluorescent dye) is prepared and the fluorescence intensity is measured at the emission peak wavelength. The emission intensity per one resin particle including fluorescent dye can be obtained by dividing the fluorescence intensity of the standard solution of resin particles including fluorescent dye by the number of the resin particles including fluorescent dye in the standard solution.

The "emission intensity of the fluorescent dye molecules alone as many as those in one resin particle including fluorescent dye" in the above definition "D" can be calculated as follows. A fixed amount of a solution (a standard solution of fluorescent dye) including a fixed concentration (for example, 0.01 nM, that is, $6.02\times10^9$ particles/mL or $W\times10^{14}$ g/mL when the molecular weight of the fluorescent dye is W) of the fluorescent dye is prepared and the fluorescence intensity is measured at the emission peak wavelength. The fluorescence intensity of the standard solution of fluorescent dye is multiplied by {(number of molecules (or weight) of the fluorescent dye included in one resin particle including fluorescent dye)/(number of molecules (or weight) of the fluorescent dye in the standard solution of fluorescent dye)} and is further multiplied by {(quantum yield of the fluorescent dye molecules alone)/(quantum yield of the resin particle including fluorescent dye)} in order to correct the influence by the concentration quenching mentioned above. The fluorescence intensity of fluorescent dye molecules alone as many as (or equivalent weight of) those included in one resin particle including fluorescent dye can be thereby obtained.

The measurement methods of emission intensity defined in above C and D are not especially limited. The measurement may be performed using a generally-used fluorophotometer in an appropriate measurement condition.

When the signal value from the solution of the resin particles including fluorescent dye satisfies the above equation, in other words, when the relative signal value, $S/\{A\times B\times(C/D)\}$, is 0.5 to 1.2, the solution of the resin particles including fluorescent dye is considered to have sufficient staining property as a staining solution. For example, the standard solution of fluorescent marker bonded to SA can be evaluated as follows.

First, the stored solution of the fluorescent marker bonded to SA is taken and the number of particles is measured by a particle counter "Liquid Particle Counter" manufactured by RION Co., Ltd.). The concentration is then adjusted to 0.05nM. After washing a commercial biotinylated plate "Well-Coated Biotin, 96-well, Black" (manufactured by G-Biosciences) with PBS five times, 100μL of the above-described solution having adjusted concentration is put in each well of the plate, followed by reaction for 24 hours at 4°C. After washing the plate after reaction with PBS, the signal value (S) of fluorescence intensity is measured using a microplate reader "Plate CHAMELEON V" (manufactured by Hidex Oy). By referring to the "immunostaining reagent D" obtained by the EXAMPLE 3 described below, for example, the relative value 0.8 was obtained by dividing the signal value (S) by [((A) signal value of the fluorescent dye molecules alone) $\times$ ((B) number of affinitive molecules bonded to one resin particle including fluorescent dye) $\times$ {((C) emission intensity per one resin particle including fluorescent dye)/((D) emission intensity of the fluorescent dye molecules alone as many as those in one resin particle including fluorescent dye)}]. It was revealed that good staining property was obtained. However, by referring to the EXAMPLE 1 described below, when the nanoparticles having accumulated fluorescent dye prepared using nikalac (melamine resin) were substituted with the nanoparticles having accumulated fluorescent dye prepared using styrene resin, the staining property was not good and the relative value of the particle was 0.4. As a whole, the staining property is good when the relative value is 0.5 or more.

According to the present invention, even in a multiple-immunostaining using particles which do not have good staining property, the accuracy of calculating the number of bright points can be improved.

### (3) ANTIBODY (MOLECULE HAVING HIGH AFFINITY TO ANTIGEN)

A primary antibody to be used includes an antibody (IgG) which specifically recognizes and bonds to an objective biological substance (protein) as an antigen. For example, anti-HER2 antibody can be used when HER2 is the objective biological substance, and anti-HER3 antibody can be used when HER3 is the objective biological substance.

A secondary antibody to be used includes an antibody (IgG) which specifically recognizes and bonds to the primary antibody.

Both primary antibody and secondary antibody may be polyclonal antibodies, but preferably monoclonal antibodies from the viewpoint of stable quantitation. Animals (immunized animals) for producing antibodies are not particularly limited, and can be selected from any conventionally used animals, such as mouse, rat, rabbit, coat, and sheep.

### (4) FLUORESCENT NANOPARTICLE

The fluorescent nanoparticle is a nano-sized particle which emits fluorescence in response to irradiation with excitation light. The fluorescent nanoparticle can emit sufficiently strong fluorescence so that each molecule of the objective biological substance is represented as a fluorescent bright point.

Preferably used fluorescent nanoparticle includes a quantum dot (a semiconductor nanoparticle) or a nanoparticle having accumulated fluorescent substance.

Preferably used fluorescent nanoparticle has an emission wavelength within the sensitivity range of the imaging element 20 of the fluorescence microscope 10, specifically, an emission wavelength of 400 to 700nm.

### (4.1) QUANTUM DOT

The quantum dot may be a semiconductor nanoparticle containing Group II-VI compounds, Group III-V compounds, or Group IV elements. Specific examples thereof include CdSe, CdS, CdTe, ZnSe, ZnS, ZnTe, InP, InN, InAs, InGaP, GaP, GaAs, Si, and Ge.

### (4.2) NANOPARTICLE HAVING ACCUMULATED FLUORESCENT SUBSTANCE

The nanoparticle having accumulated fluorescent substance is a nano-sized particle containing an organic or inorganic particle as a base material and further having a plurality of fluorescent substance molecules (for example, above-described quantum dots and fluorescent dyes) in the particle and/or on the surface of the particle.

The base material and the fluorescent substance of the nanoparticle having accumulated fluorescent substance respectively include a substituent group or a site having opposite electric charge from each other. An electrostatic interaction is thereby preferably caused.

The nanoparticle having accumulated fluorescent substance includes a nanoparticle having accumulated quantum dots, a nanoparticle having accumulated fluorescent dye, and the like.

### (4.2.1) BASE MATERIAL

Examples of an organic base material include resins generally classified into thermosetting resins, such as melamine resins, urea resins, aniline resins, guanamine resins, phenol resins, xylene resins, and furan resins; resins generally classified into thermoplastic resins, such as styrene resins, acrylic resins, acrylonitrile resins, AS resins (acrylonitrile- styrene copolymer resin), and ASA resins (acrylonitrile-styrene-acrylic resins); other resins such as polylactic acid; and polysaccharides.

Examples of an inorganic base material include silica, glass, and the like.

### (4.2.2) NANOPARTICLE HAVING ACCUMULATED QUANTUM DOTS

The nanoparticle having accumulated quantum dots contains the above-described quantum dots in the base material and/or adsorbed on the surface of the base material.

If the quantum dots are in the base material, the quantum dots may be dispersed within the base material in any form. The quantum dots and the base material may or may not be chemically bonded with each other.

### (4.2.3) NANOPARTICLE HAVING ACCUMULATED FLUORESCENT DYE

The nanoparticle having accumulated fluorescent dye contains fluorescent dye in the base material and/or adsorbed on the surface of the base material.

Examples of the fluorescent dye include rhodamine-based dye molecules, squarylium-based dye molecules, cyanine-based dye molecules, aromatic ring-based dye molecules, oxazine-based dye molecules, carbopyronine-based dye molecules, and pyrromethene-based dye molecules.

Examples of the fluorescent dye include Alexa Fluor (registered trademark, made by Invitrogen Corporation) dye molecules, BODIPY (registered trademark, made by Invitrogen Corporation) dye molecules, Cy (registered trademark, made by GE Healthcare) dye molecules, HiLyte (registered trademark, made by AnaSpec Inc.) dye molecules, DyLight (registered trademark, made by Thermo Scientific Inc.) dye molecules, ATTO (registered trademark, made by ATTO-TEC GmbH.) dye molecules, MFP (registered trademark, made by Mobitec Inc.) dye molecules, CF (registered trademark, made by Biotium Inc.) dye molecules, DY (registered trademark, made by Dyomics GmbH) dye molecules, CAL (registered trademark, made by BioSearch TechnologiesInc.) dye molecules, and the like.

If the fluorescent dye is included in the base material, the fluorescent dye may be dispersed within the base material in any form. The fluorescent dye and the base material may or may not be chemically bonded with each other.

### (5) STAINING METHOD OF TISSUE SLICE

An exemplary staining method will be explained.

A tissue slice (hereinafter may be simply referred to as a "slice" and includes a slice such as a pathological slice) prepared by any known procedures can be applied to the staining method described below.

### (5.1) PREPARATION STEP OF SPECIMEN

### (5.1.1) DEPARAFFINIZATION PROCESS

A slice is immersed in a container containing xylene so that paraffin is removed. The temperature is not particularly limited, and the process can be performed at room temperature. Preferably, the immersing time is 3 minutes or more and 30 minutes or less. The xylene may be changed during the immersion as needed.

Next, the slice is immersed in a container containing ethanol so that the xylene is removed. The temperature is not particularly limited, and the process can be performed at room temperature. Preferably, the immersing time is 3 minutes or more and 30 minutes or less. The ethanol may be changed during the immersion as needed.

The slice is immersed in a container containing water so that the ethanol is removed. The temperature is not particularly limited, and the process can be performed at room temperature. Preferably, the immersing time is 3 minutes or more and 30 minutes or less. The water may be changed during the immersion as needed.

### (5.1.2) RETRIEVAL PROCESS

In accordance with a publically-known method, the objective biological substance is subjected to retrieval process. The retrieval process can be performed under any condition. As for a retrieving solution, a 0.01 M citrate buffer solution (pH 6.0), a 1 mM EDTA solution (pH 8.0), 5% urea, a 0.1 M Tris-hydrochloride buffer solution, and the like can be used.

The retrieval process is performed under a condition of pH 2.0 to 13.0 depending on the kind of the tissue slice, so that a signal is emitted and the damage of the tissue does not prevent evaluation of the signal from the tissue. The retrieval process is usually performed at pH 6.0 to 8.0, but is performed at pH 3.0, for example, in the case of a special tissue slice.

As for a heater, an autoclave, a microwave heater, a pressure cooker, a water bath, or the like can be used. The temperature is not particularly limited, and the process can be performed at room temperature. The temperature may range from 50 to 130°C, and the time may range from 5 to 30 minutes.

Subsequently, the slice after the retrieval process is immersed in a container including PBS and washed. The temperature is not particularly limited, and the process can be performed at room temperature. Each immersion time is preferably 3 minutes or more and 30 minutes or less. The PBS may be changed during the immersion as needed.

### (5.2) IMMUNOSTAINING STEP

In the immunostaining step for staining the objective biological substance, a solution of immunostaining reagent is put on the slice, and the objective biological substance reacts with the fluorescent nanoparticles in the immunostaining reagent including a site which can directly or indirectly bond to the objective biological substance. The solution of the immunostaining reagent used in the staining step can be prepared in advance before the step.

The conditions for the immunostaining step, i.e. the temperature and time of immersing the tissue specimen in the solution of the immunostaining reagent, can be suitably adjusted according to the conventional immunostaining method, so that appropriate signals can be obtained.

The temperature is not particularly limited, and the processing can be performed at room temperature. Preferably, the immersing time is 30 minutes or more and 24 hours or less.

Before performing the above-described processing, it is preferable to add a known blocking reagent such as PBS including BSA and a surfactant such as Tween 20.

## (5.3) POST-PROCESSING STEP OF SPECIMEN

After the immunostaining step, the tissue specimen is preferably subjected to processes such as fixation-dehydration, permeation, and encapsulation.

The fixation-dehydration process is performed by immersing the tissue specimen in a solution for fixation processing (crosslinking reagent such as formalin, paraformaldehyde, glutaraldehyde, acetone, ethanol, and methanol). The permeation process is performed by immersing the tissue specimen after the fixation-dehydration process in a solution for permeation (such as xylene). The encapsulation process is performed by immersing the tissue specimen after the permeation process in a solution for encapsulation.

The conditions for these processes, for example, the temperature and time of immersing the tissue specimen in the prescribed solution, can be suitably adjusted so that appropriate signals can be obtained, according to the conventional immunostaining method.

## (5.4) STAINING STEP FOR MORPHOLOGICAL OBSERVATION

Apart from the immunostaining step, staining for morphological observation may be performed for observing morphology of a cell, tissue, and organ in a bright field image.

The staining step for morphological observation can be performed according to a usual method.

For morphological observation of the tissue specimen, staining by eosin is generally performed for staining cytoplasm, stroma, various fibers, red blood cell, and keratinocyte in red to dark red. Staining by hematoxylin is also generally performed for staining a cell nucleus, calcification portion, cartilage, bacteria, and mucus in livid to light blue. (The method to perform these two stainings simultaneously is known as hematoxylin-eosin staining (HE staining).)

The staining step for morphological observation may be after or before the immunostaining step.

## [ANALYSIS METHOD OF A TARGET BIOLOGICAL SUBSTANCE]

Next, analysis method of a target biological substance will be explained.

According to the method, the tissue specimen is stained with immunostaining reagents including fluorescent nanoparticles different from each other and analyzed with the analysis system 1 of a target biological substance.

As shown in FIG. 2, the main steps of the analysis method of the target biological substance are as follows:

(S1) Step S1 to calculate luminance ratio of each immunostaining reagent for each filter set 50 (hereinafter, also referred to as "luminance ratio calculation step S1")

(S2) Step S2 to measure the number of fluorescent bright points in tissue specimen 30 for each filter set 50 and to calculate the number of fluorescent bright points for each immunostaining reagent on the basis of the luminance ratio (hereinafter, also referred to as "number of fluorescent bright point calculation step S2")

## (1) LUMINANCE RATIO CALCULATION STEP S1

In the luminance ratio calculation step S1, a sample scattered with multiple kinds of immunostaining reagents and multiple kinds of filter sets 50 are prepared corresponding to the number of kinds of target biological substance for calculating luminance ratio.

The "sample scattered with immunostaining reagents" is a glass substrate on which immunostaining reagents are applied. The "multiple kinds of immunostaining reagents" includes fluorescent nanoparticles different from each other corresponding to the number of kinds of target biological substance in the tissue specimen sample. The "multiple kinds of filter sets 50" includes excitation filters 52, beam splitters 54, and fluorescence filters 56, different from each other and also corresponding to the number of kinds of target biological substance in the tissue specimen sample.

Subsequently, the sample scattered with immunostaining reagents is put on the stage 12.

Subsequently, while switching the filter sets 50, every time the filter sets 50 are switched, the control unit 62 turns on the lamp 40, controls the imaging element 20 to image a fluorescent image of the tissue specimen sample, and measures the number of fluorescent bright points in the tissue specimen sample for each filter set 50.

In measuring the number of fluorescent bright points, image processing software such as "ImageJ" (open source) can be used so that fluorescent bright points having a predetermined wavelength (color) in a fluorescent image can be extracted and measured semi-automatically and rapidly.

Subsequently, on the basis of the measurement result of the number of fluorescent bright points for each filter set 50, the control unit 62 calculates luminance ratio of each immunostaining reagent for each filter set 50 and makes the storage 64 to store the luminance ratio.

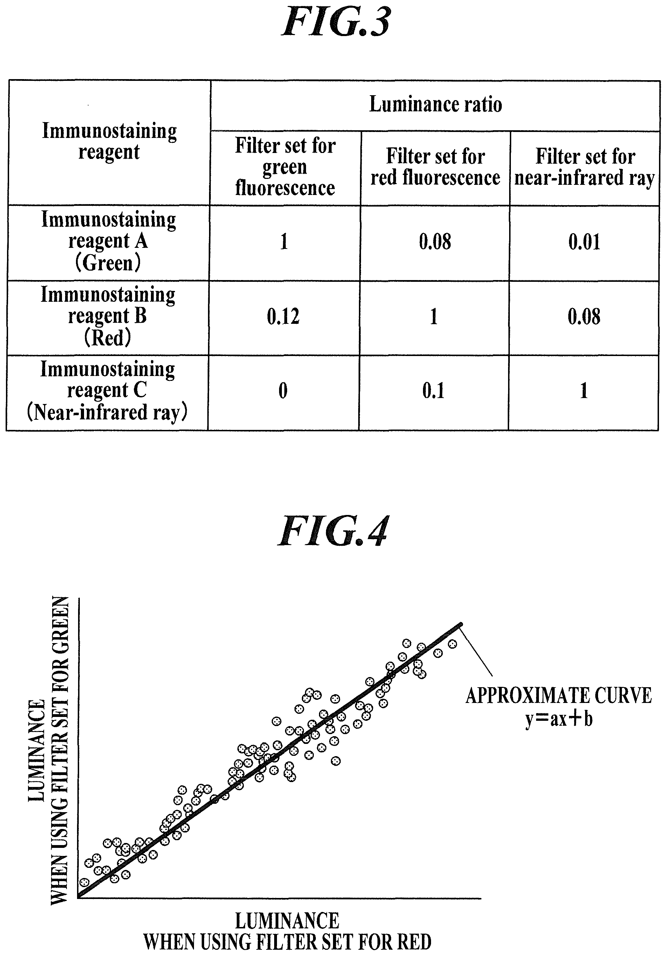

For example, as shown in FIG. 3, three kinds of immunostaining reagents A to C are used, respectively including fluorescent nanoparticles emitting fluorescence of green, red, and near-infrared ray. While switching three kinds of filter sets 50, respectively for green, red, and near-infrared ray, every time the filter sets 50 are switched, the number of fluorescent bright points in the fluorescent image of the tissue specimen sample is measured and the luminance ratio of each immunostaining reagent is calculated for each filter set 50.

It is shown in FIG. 3 that, as for the immunostaining reagent A including fluorescent nanoparticles emitting green fluorescence, the luminance ratio is calculated to be 1:0.08:0.01 when numbers of fluorescent bright points are measured using filter sets 50, respectively for green, red, and near-infrared ray.

As for the immunostaining reagent B including fluorescent nanoparticles emitting red fluorescence, the luminance ratio of is calculated to be 0.12:1:0.08 when numbers of fluorescent bright points are measured using filter sets 50, respectively for green, red, and near-infrared ray.

As for the immunostaining reagent C including fluorescent nanoparticles emitting near-infrared ray, the luminance ratio of is calculated to be 0:0.1:1 when numbers of fluorescent bright points are measured using filter sets 50, respectively for green, red, and near-infrared ray.

The specific examples of the calculation method of the luminance ratio include the following two methods. According to the first method (first calculation method), the total luminance value of the fluorescent bright points measured in the entire photographed image is used. The ratio of the total luminance values is determined to be the luminance ratio. This method is easily performed by averaging the entire image and can be advantageously used especially when each particle does not have a characteristic luminance ratio, that is, each luminance ratio is not biased to a characteristic value.

The specific example of the first calculation method includes calculating luminance ratio R(1 to n, 1 to N) from the following General formula (1).

$$\text{General formula (1): luminance ratio } R(m, k) = FL(m, k)/FL(m, p),$$

(In the above General formula (1), FL(m, k) represents a total luminance value of fluorescent bright points observed in an entire image of an immunostaining reagent(m) using a filter set(k), FL(m, k) represents a total luminance value of fluorescent bright points observed in an entire image of the immunostaining reagent(m) using a filter set(p), k represents an integer of 1 to N, p represents an integer of 1 to N, and n, N, and m respectively represent a positive integer and satisfy the expression of $1 \leq m \leq n$.

In the above General formula (1), m, k, and p preferably satisfy the expression of $FL(m, k) \leq FL(m, p)$, because the upper limit of the luminance ratio R(m, k) is 1 according to calculations. That is, among the filter sets(1 to N), the filter set(p) is preferably the one providing the maximum total luminance value of bright points observed in an entire image of the immunostaining reagent(m).

Meanwhile, according to the second method (second calculation method), the luminance value per one bright point is measured for each filter set 50 and the luminance ratio is calculated on the basis of the luminance values for each filter set. Specifically, for example, in order to obtain the luminance ratio of the immunostaining reagent A including fluorescent nanoparticles emitting green fluorescence on the basis of the luminance values measured using filter sets 50, respectively for green, red, and near-infrared ray, a graph is prepared. The graph shows a luminance value measured with the filter set 50 for green on the vertical axis and a luminance value measured with the filter set 50 for red (or near-infrared ray) on the horizontal axis (see FIG. 4). An approximate curve (y = ax) is obtained from the graph and the "a" is determined to be the luminance ratio of the measured value using the filter set for green to the measured value using the filter set 50 for red(or near-infrared ray).

That is, according to the second calculation method, the luminance value of the same fluorescent bright point is measured for each of the filter sets(1 to N) at first. The luminance ratio R(1 to n, 1 to N) is determined as a luminance ratio R(m, k), which is an inclination a described in the following General formula (2). The General formula (2) represents an approximate straight line indicating the relation between a luminance value X obtained using a filter set(p) and a luminance value Y obtained using a filter set(k). According to the second calculation method, k represents an integer of 1 to N, p represents an integer of 1 to N, and n, N, and m respectively represent a positive integer and satisfy the expression of $1 \leq m \leq n$.

$$\text{General formula (2): } Y = aX$$

In the above General formula (2), among the filter sets(1 to N), the filter set(p) is preferably the one providing the maximum total luminance value of bright points observed in an entire image of the immunostaining reagent(m) because the upper limit of "a" is 1 according to calculations.

According to the second calculation method, even when each molecule has a characteristic luminance ratio, that is, each luminance ratio is biased to a characteristic value, the luminance ratio can be close to a true luminance ratio.

(2) NUMBER OF FLUORESCENT BRIGHT POINT CALCULATION STEP S2

In the number of fluorescent bright point calculation step S2, the tissue specimen 30 is stained by the multiple immunostaining reagents as described above. The tissue specimen 30 is put on the stage 12 after immunostaining.

Subsequently, while switching the filter sets 50, every time the filter sets 50 are switched, the control unit 62 turns on the lamp 40, controls the imaging element 20 to image a fluorescent image, and measures the number of fluorescent bright points in the tissue specimen 30 for each filter set 50.

Subsequently, the control unit 62 reads out the luminance ratio calculated in the luminance ratio calculation step S1 and stored in the storage 64. On the basis of the luminance ratio, the number of fluorescent bright points of each immunostaining reagent is calculated.

In such cases, number of fluorescent bright points L(m) for the immunostaining reagent(m) is calculated from the solution of the following simultaneous equations:

$$TB(1) = L(1) + R(1,2) \times L(2) + R(1,3) \times L(3) + \cdots$$
$$TB(2) = R(2,1) \times L(1) + L(2) + R(2,3) \times L(3) + \cdots$$
$$TB(3) = R(3,1) \times L(1) + R(3,2) \times L(2) + L(3) + \cdots$$
$$\cdots\cdots\cdots\cdots\cdots$$

In the equations, R(1 to n, 1 to N) represents luminance ratio of each immunostaining reagent(1 to n) for each of the filter sets 50(1 to N), TB(1 to N) represents number of fluorescent bright points in the tissue specimen 30 measured for each of the filter sets 50(1 to N), L(1 to n) represents number of fluorescent bright points of each of the immunostaining reagents(1 to n), and n, N, and m respectively represent a positive integer and satisfy the expression of $1 \leq m \leq n$.

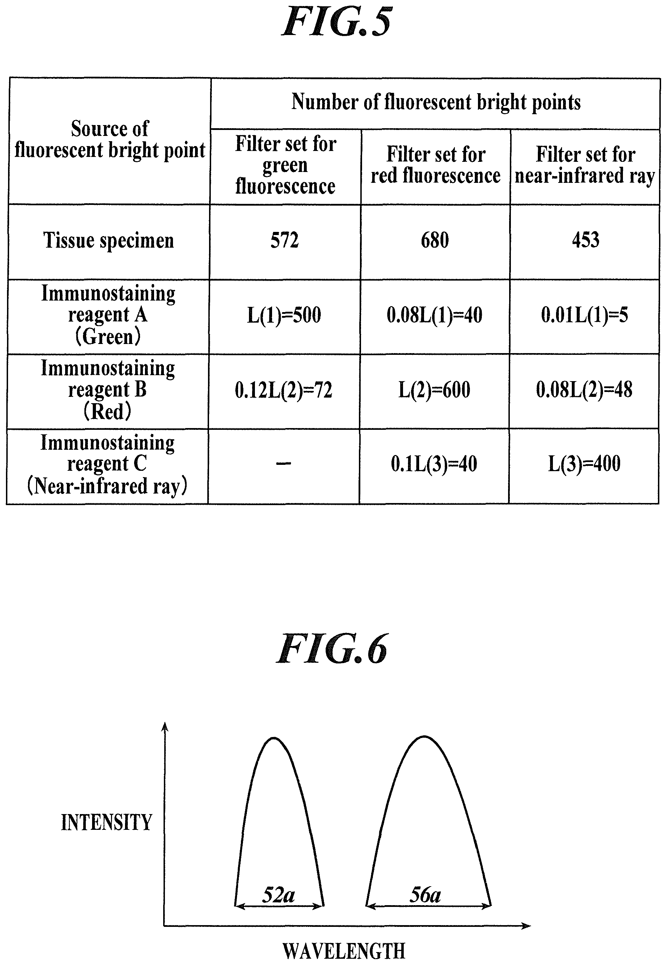

For example, FIG. 5 shows a case in which the tissue specimen 30 includes three kinds of target biological substances and three kinds of immunostaining reagents A to C are used for staining, respectively including fluorescent nanoparticles that emit fluorescence of green, red, and near-infrared ray. While switching the three kinds of filter sets 50, respectively for green, red, and near-infrared ray, every time the filter sets 50 are switched, the number of fluorescent bright points in the fluorescent image of the tissue specimen 30 is measured.

In such cases, if the measured fluorescent bright points are 572 using the filter set for green, 680 using the filter set for red, and 453 using the filter set for near-infrared ray, the following simultaneous equations can be obtained:

$$572 = L(1) + 0.12L(2) + 0L(3)$$

$$680 = 0.08L(1) + L(2) + 0.1L(3)$$

$$453 = 0.01L(1) + 0.08L(2) + L(3)$$

The solutions of the simultaneous equations are L(1) = 500, L(2) = 600, and L(3) = 400. Accordingly, the numbers of fluorescent bright points are calculated to be 500 for green, 600 for red, and 400 for near-infrared ray. As a result, the number of fluorescent bright points can be calculated for each of the immunostaining reagents A to C.

In the luminance ratio calculation step S1 and the number of fluorescent bright point calculation step S2, the filter sets 50 preferably include, as shown in FIG. 6, an excitation filter 52 having a wavelength width 52a of 20 to 30 nm and a fluorescence filter 56 having a wavelength width 56a of 30 to 50 nm.

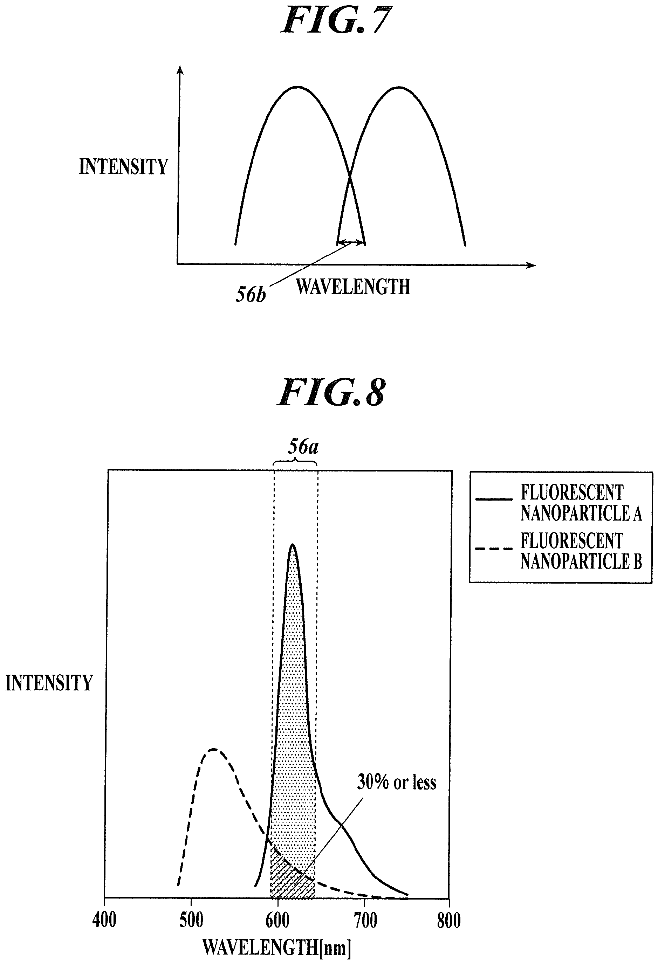

In such cases, as shown in FIG. 7, the overlapping wavelength width 56b of fluorescence filters 56 is preferably 10 nm or less in the filter sets 50.

The overlapping emission wavelength of the fluorescent nanoparticles is preferably by 30% or less in the immunostaining reagents.

The "overlapping emission wavelength" is, as shown in FIG. 8, overlapping of the fluorescent spectrum of fluorescent nanoparticle A and that of another fluorescent nanoparticle B. More specifically, the integrated intensity value of the fluorescent nanoparticle B (see hatched line portion) relative to the integrated intensity value of the fluorescent nanoparticle A (see gray portion) within the wavelength width 56a of the fluorescence filter 56 corresponding to the fluorescent nanoparticle A. According to the example shown in FIG. 8, the area of the hatched line portion relative to the gray portion is preferably 30% or less when using the fluorescent nanoparticle B.

According to the above embodiments, the luminance ratio of each immunostaining reagent is calculated for each filter set 50 in advance. Subsequently, the number (total number) of fluorescent bright points is measured for each filter set 50, and the number of fluorescent bright points for each immunostaining reagent is calculated on the basis of the calculated luminance ratio.

In such embodiments, during fluorescence observation using one filter set 50, number of fluorescent bright points for the immunostaining reagent of the fluorescence observation target can be calculated based on the luminance ratio of having already been calculated, even when fluorescence of other immunostaining reagents is also observed. Accordingly, even in multiple-immunostaining using multiple kinds of immunostaining reagents, the number of fluorescent bright points for each immunostaining reagent can be accurately analyzed.

EXAMPLES

[EXAMPLE 1]

(1) PREPARATION OF IMMUNOSTAINING REAGENT

(1.1) SYNTHESIS OF NANOPARTICLES HAVING ACCUMULATED FLUORESCENT DYE

14.4 mg of Pyrromethene 556 dye, which is a green light emitting dye used as a fluorescent dye, was dissolved in 22ml of water. To the solution was added 2ml of 5% aqueous solution of Emulsion (registered trademark) 430 (polyoxyethylene oleyl ether, manufactured by Kao Corporation), which is an emulsion for emulsion polymerization. After heating to 70°C during stirring on a hot stirrer, 0.65 g of Nikalac MX-035 as a raw material of melamine resin (manufactured by Nippon Carbide Industries Co., Inc.) was added to the solution.

Next, 1000 μL of 10% aqueous solution of dodecylbenzenesulfonic acid (manufactured by Kanto Chemical Co., Inc.) as a reaction initiator was added to the solution and was heated and stirred for 50 minutes at 70°C. Thereafter, the solution was further heated and stirred for 20 minutes after heating to 90°C.

The obtained particle dispersion liquid of dye and resin was washed with pure water in order to remove impurities such as excess raw materials of resin and fluorescent dye. Specifically, the dispersion was centrifuged at 20,000 G for 15 minutes with a Centrifugal separator (Micro Refrigerated Centrifuge 3740 manufactured by Kubota Corp.), subjected to removal process of supernatant, and subjected to re-dispersion process by addition of extra pure water and ultrasonic irradiation. The centrifugation, removal process, and re-dispersion process were repeated five times.

Nanoparticles having accumulated green fluorescent dye (excitation wavelength was 490 nm and emission wavelength was 520 nm) were thereby prepared.

Nanoparticles having accumulated red fluorescent dye (excitation wavelength was 590 nm and emission wavelength was 620 nm) were also prepared as in the preparation of the nanoparticles having accumulated green fluorescent dye, except that Sulforhodamine 101 (Texas Red) dye was used instead of the Pyrromethene 556 dye.

Nanoparticles having accumulated near infrared dye (excitation wavelength was 643 nm, emission wavelength was 647 nm) were also prepared as in the preparation of the nanoparticles having accumulated green fluorescent dye, except that Cy5 dye was used instead of the Pyrromethene 556 dye.

(1.2) MODIFICATION OF NANOPARTICLE SHAVING ACCUMULATED FLUORESCENT DYE

At the end of the nanoparticles having accumulated green fluorescent dye, maleimide was introduced using NHS-PEG (polyethylene glycol)-maleimide reagent. A thiolated anti-HER2 antibody was bonded thereto, to prepare "immunostaining reagent A."

In the same way, at the end of the nanoparticles having accumulated red fluorescent dye, maleimide was introduced and a thiolated anti-Ki67 antibody was bonded thereto, to prepare "immunostaining reagent B."
At the end of the nanoparticles having accumulated near infrared dye, maleimide was introduced and a thiolated anti-Cytokeratin 14 antibody was bonded thereto, to prepare "immunostaining reagent C."

(2) PREPARATION OF SAMPLE SCATTERED WITH IMMUNOSTAINING REAGENTS AND MEASUREMENT OF LUMINANCE RATIO

(2.1) A slide scattered with immunostaining reagents was prepared by applying the immunostaining reagents A to C respectively on a glass slide. A sample of human breast tissue specimen may be used, if it includes three kinds of antigens, HER2, Ki67, and Cytokeratin 14, and the ratio of the expressing antigens is known in advance.

(2.2) OBSERVATION WITH FLUORESCENCE MICROSCOPE

A fluorescence microscope manufactured by Carl Zeiss AG and filter sets manufactured by Semrock were used. Three kinds of filter sets were used corresponding to the immunostaining reagents A to C (for green, red, and near-infrared ray).

TABLE 1

| Filter set | Excitation wavelength / emission wavelength of filter set | | |
|---|---|---|---|
| | For green (For Pyromethene 556) | For or red (For Texas Red) | For near-infrared ray (For Cy5) |
| Excitation filter | 470nm (Wavelength width 30nm) | 586nm (Wavelength width 20nm) | 640nm (Wavelength width 30nm) |
| Beam splitter | 495nm | 605nm | 660nm |
| Fluorescence filter | 525nm (Wavelength width 50nm) | 628nm (Wavelength width 32nm) | 690nm (Wavelength width 50nm) |

(3) OBSERVATION WITH FLUORESCENCE MICROSCOPE

The sample scattered with immunostaining reagents was put on the stage. While switching the three kinds of filter sets, respectively for green, red, and near-infrared ray, every time the filter sets were switched, the luminance ratio was calculated as the ratio of the total luminance values of the fluorescent bright points in the entire screen of the fluorescent image of the sample scattered with immunostaining reagents (according to the first calculation method) or as the ratio of the immunostaining reagents A to C for each filter set based on the luminance value per one bright point (according to the second calculation method). The same luminance ratio was obtained by either the first calculation method or the second calculation method.

TABLE 2

| Immunostaining reagent | Luminance ratio | | |
|---|---|---|---|
| | Filter set for green fluorescence | Filter set for red fluorescence | Filter set for near-infrared ray |
| Immunostaining reagent A (Green) | 1 | 0.08 | 0.01 |
| Immunostaining reagent B (Red) | 0.12 | 1 | 0.08 |
| Immunostaining reagent C (Near-infrared ray) | 0 | 0.1 | 1 |

(4) IMMUNOSTAINING OF TISSUE SPECIMEN

Immunostaining of a human breast tissue specimen was performed.

Immunostaining (IHC method) of the human breast tissue specimen was performed according to the following steps (4-1) to (4-15) using the immunostaining reagents A to C.

Step (4-1): The tissue specimen was immersed in a container containing xylene for 15 minutes. The xylene was changed twice during the immersion.

Step (4-2): The tissue specimen was immersed in a container containing ethanol for 10 minutes. The ethanol was changed twice during the immersion.

Step (4-3): The tissue specimen was immersed in a container containing water for 10 minutes.

Step (4-4): The tissue specimen was immersed in 10 mM citric acid buffer solution (pH 6.0).

Step (4-5): Autoclaving was performed for 5 minutes at 121°C.

Step (4-6): The tissue specimen after autoclaving was immersed in a container containing PBS for 15 minutes. The PBS was changed three times during the immersion.

Step (4-7): 1% BSA-containing PBS was put on the tissue specimen and left for 1 hour.

Step (4-8): The immunostaining reagent A with anti-HER2 antibody was diluted to 0.1 nM with 1% BSA-containing PBS and put on the tissue specimen and left overnight.

Step (4-9): The tissue specimen after staining was immersed in a container containing PBS for 15 minutes.

Step (4-10) The immunostaining reagent B with anti-Ki67 antibody was diluted to 0.1nM with 1% BSA-containing PBS and put on the tissue specimen and left overnight.

Step (4-11): The tissue specimen after staining was immersed in a container containing PBS for 15 minutes.

Step (4-12): The immunostaining reagent C with anti-Cytokeratin 14 antibody was diluted to 0.1nM with 1% BSA-containing PBS and put on the tissue specimen and left overnight.

Step (4-13): The tissue specimen after staining was immersed in a container containing PBS for 30 minutes.

In the steps (4-8) to (4-13), the immunostaining reagents A, B, and C having an final concentration of 0.1 nM adjusted by 1% BSA-containing PBS may be put on the tissue specimen and left overnight, followed by immersion for 15 minutes in a container containing PBS.

Step (4-14): After fixation with 4% neutral paraformaldehyde solution for 10 minutes, the tissue specimen was subjected to HE staining.

Step (4-15): Aquatex, manufactured by Merck Chemicals, was dropped thereon and then a cover glass was placed for sealing the tissue specimen.

(5) MICROSCOPIC OBSERVATION

The tissue specimen after immunostaining was put on the stage. While switching the three kinds of filter sets 50, respectively for green, red, and near-infrared ray, every time the filter sets were switched, the number of fluorescent bright points in the fluorescent image of the tissue specimen was measured

TABLE 3

| Source of fluorescent bright point | Number of fluorescent bright points | | |
|---|---|---|---|
| | Filter set for green fluorescence | Filter set for red fluorescence | Filter set for near-infrared ray |
| Tissue specimen | 1656 | 1545 | 1369 |
| Immunostaining reagent A (Green) | L(1)=1500 | 0.08L(1)=120 | 0.01L(1)=15 |
| Immunostaining reagent B (Red) | 0.12L(2)=156 | L(2)=1300 | 0.08L(2)=104 |
| Immunostaining reagent C (Near-infrared ray) | - | 0.1L(3)=125 | L(3)=1250 |

On the basis of the measurement result of the number of fluorescent bright points, the following simultaneous equations were obtained:

$$1656 = L(1) + 0.12L(2) + 0L(3)$$

$$1545 = 0.08L(1) + L(2) + 0.1L(3)$$

$$1369 = 0.01L(1) + 0.08L(2) + L(3)$$

The solutions of the simultaneous equations were L(1) = 1500, L(2) = 1300, and L(3) = 1250. Accordingly, the numbers of fluorescent bright points were calculated to be 1500 for green, 1300 for red, and 1250 for near-infrared ray. As a result, the number of fluorescent bright points could be calculated for each of the immunostaining reagents A to C.

From the above-described results, it was confirmed that the tissue specimen to be examined expressed HER2, Ki67, and Cytokeratin 14 at the ratio of HER2:Ki67:Cytokeratin14 = 1500:1300:1250.

[EXAMPLE 2]

(1) PREPARATION OF IMMUNOSTAINING REAGENT

(1.1) SYNTHESIS OF NANOPARTICLES HAVING ACCUMULATED FLUORESCENT DYE

Nanoparticles having accumulated green fluorescent dye (excitation wavelength was 490 nm, emission wavelength was 520 nm) was prepared as in the "(1.1) SYNTHESIS OF NANOPARTICLES HAVING ACCUMULATED FLUORESCENT DYE" of EXAMPLE 1.

Nanoparticles having accumulated red fluorescent dye (excitation wavelength was 590 nm, emission wavelength was 620 nm) were also prepared as in the preparation of the nanoparticles having accumulated green fluorescent dye, except that Sulforhodamine 101 (Texas Red) dye was used instead of the Pyrromethene 556 dye.

(1.2) MODIFICATION OF NANOPARTICLES HAVING ACCUMULATED FLUORESCENT DYE

At the end of the nanoparticles having accumulated green fluorescent dye, maleimide was introduced using NHS-PEG (polyethylene glycol)-maleimide reagent. A thiolated anti-Digoxigenin (DIG) antibody was bonded thereto, to prepare "immunostaining reagent D."

In the same way, at the end of the nanoparticles having accumulated red fluorescent dye, maleimide was introduced and a thiolated anti-Fluorescein (FITC) antibody was bonded thereto, to prepare "immunostaining reagent E."

(1.3) MODIFICATION OF SECONDARY ANTIBODY

NHS-DIG was bonded to the amino group of rat-derived anti-mouse antibody SB77e to prepare "secondary antibody A."

In the same way, NHS-FITC was bonded to rat-derived anti-rabbit antibody LORG1 to prepare "secondary antibody B."

(2) PREPARATION OF SAMPLE SCATTERED WITH IMMUNOSTAINING REAGENTS AND MEASUREMENT OF LUMINANCE RATIO

A slide scattered with immunostaining reagents was prepared by applying the immunostaining reagents D and E on a glass slide.

(3) OBSERVATION WITH FLUORESCENCE MICROSCOPE

The sample scattered with immunostaining reagents was put on the stage. While switching the two kinds of filter sets, respectively for green and red, every time the filter sets were switched, the total luminance value of the fluorescent bright points in the entire screen of the fluorescent image of the tissue specimen sample or the luminance value per one bright point was measured. The luminance ratio of immunostaining reagents D and E were respectively thereby calculated for each filter set.

(4) IMMUNOSTAINING OF TISSUE SPECIMEN

Immunostaining (IHC method) of a human lung tissue specimen was performed according to the following steps (4-1) to (4-15) using the immunostaining reagents D and E.

Step (4-1): The tissue specimen was immersed in a container containing xylene for 15 minutes. The xylene was changed twice during the immersion.

Step (4-2): The tissue specimen was immersed in a container containing ethanol for 10 minutes. The ethanol was changed twice during the immersion.

Step (4-3): The tissue specimen was immersed in a container containing water for 10 minutes.

Step (4-4): The tissue specimen was immersed in 10 mM citric acid buffer solution (pH 6.0).

Step (4-5): Autoclaving was performed for 5 minutes at 121°C.

Step (4-6): The tissue specimen after autoclaving was immersed in a container containing PBS for 15 minutes. The PBS was changed three times during the immersion.

Step (4-7): 1% BSA-containing PBS was put on the tissue specimen and left for 1 hour.

Step (4-8): A mixed solution of PD-L1 antibody and anti-EGFR antibody was diluted to 3 $\mu$g/ml with 1% BSA-containing PBS, put on the tissue specimen, and left overnight.

Step (4-8): A mixed solution of the secondary antibody A bonded with DIG and the secondary antibody B bonded with FITC was diluted to 2 $\mu$g/ml with 1% BSA-containing PBS, put on the tissue specimen, and left overnight.

Step (4-8): The immunostaining reagent D bonded with anti-DIG antibody and the immunostaining reagent E bonded with anti-FITC antibody were diluted to 0.1nM with 1% BSA-containing PBS, put on the tissue specimen, and left overnight.

Step (4-13): The tissue specimen after staining was immersed in a container containing PBS for 30 minutes.

Step (4-14): After fixation with 4% neutral paraformaldehyde solution for 10 minutes, the tissue specimen was subjected to HE staining.

Step (4-15): Aquatex, manufactured by Merck Chemicals, was dropped thereon and then a cover glass was placed for sealing the tissue specimen.

(5) MICROSCOPIC OBSERVATION

A fluorescence microscope manufactured by Carl Zeiss AG and filter sets manufactured by Semrock were used. Two kinds of filter sets were used corresponding to the immunostaining reagents D and E (for green and red). The microscopic observation and the analysis of fluorescent bright points were performed as in the "(5) MICROSCOPIC OBSERVATION" of EXAMPLE 1.

Subsequently, the luminance ratio was calculated from the total luminance values of the fluorescent bright points in the entire screen of the fluorescent screen (according to the first calculation method). Furthermore, the luminance ratio of each of immunostaining reagents A to C for each filter set was calculated from the measured luminance value per bright point (according to the second calculation method). The same luminance ratios (TABLE 4) were obtained by either the first calculation method or the second calculation method. It was confirmed that the expression ratio was PD-L1:EGFR = 200:1400 (TABLE 5).

TABLE 4

| Source of fluorescent bright point | Luminance ratio | |
|---|---|---|
| | Filter set for green fluorescence | Filter set for red fluorescence |
| Immunostaining reagent D (Green) | 1 | 0.08 |
| Immunostaining reagent E (Red) | 0.12 | 1 |

TABLE 5

| Source of fluorescent bright point | Luminance ratio | |
|---|---|---|
| | Filter set for green fluorescence | Filter set for red fluorescence |
| Tissue specimen | 1424 | 312 |
| Immunostaining reagent D(Green) | L(1)=1400 | 0.08L(1)=112 |
| Immunostaining reagent E (Red) | 0.12L (2) =24 | L(2)=200 |

[EXAMPLE 3 (EXAMPLE IN WHICH TISSUE SPECIMEN WAS COLLECTED FROM A MODEL ANIMAL AND PROCESSED TO BE A SPECIMEN)]

Bright points derived from PD-L1 and EGFR were detected as in EXAMPLE 2, except that the tissue specimen to be examined was a specimen of tissue collected from a PDX mouse (Patient-derived xenograft).

PDX is an abbreviated name of Patient-derived tumor xenograft, by which tissue derived from a patient (human) grafted to a mouse or other experimental animal grows in the body of mouse etc. for a prescribed period. For example, it has been recently discussed that tissue of human cancer grafted to a mouse increases during rearing of the mouse for about one month and that it is useful as a substitute for a specimen from a patient. Grafting the increased cancer tissue to yet another mouse is referred to as "passage" and the specimen of a patient can continuously increase by passage.

The tissue specimen used in this example was prepared by the following procedure.

(1) ACQUISITION OF SPECIMEN: Specimen to be grafted was a lung cancer patient having an area of 1 cm$^3$ purchased from a supplier company of clinical specimen, Sofia Bio LLC.

(2) PREPARATION OF PDX MOUSE: 3 mm$^3$ of the obtained specimen of cancer tissue was subcutaneously grafted to three NOD-SCID mice, respectively. After rearing the mice in a sterile facility for about one month, the cancer tissue increased to 1 cm$^3$ was collected. A part of the collected cancer tissue was fixed by 10% formalin for 24 hours and stored as a block using paraffin.

(3) STAINING OF SLIDE OF PARAFFIN SLICE

A slice having a thickness of 4 $\mu$m cut out from the obtained paraffin block was stuck to a glass slide. Immunostaining and analysis of a stained image were performed as in EXAMPLE 2. The "(1.2) MODIFICATION OF NANOPARTICLE HAVING ACCUMULATED FLUORESCENT DYE" in EXAMPLE 2 was changed as follows.

At the end of the nanoparticles having accumulated green fluorescent dye, maleimide was introduced using NHS-PEG (polyethylene glycol)-maleimide reagent. Melamine nanoparticles including fluorescent substance having a maleimide group at the end were thereby obtained. Meanwhile, a thiol group was added to streptavidin (made by Wako Pure Chemical Industries, Ltd.) using N-succinimidyl S-acetylthioacetate (SATA). By subsequent filtration through a gel filtration column, a solution of streptavidin bindable to the melamine nanoparticles including fluorescent substance was prepared. The melamine nanoparticles including fluorescent substance and the streptavidin were mixed in 2 mM of EDTA in PBS and reacted for one hour at room temperature. 10 mM of mercaptoethanol was added to terminate the reaction. The obtained solution was condensed with a centrifugal filter, and was passed through a gel filtration column for purification to remove unreacted streptavidin and other impurities. Melamine nanoparticles including fluorescent substance and binding to streptavidin were thereby prepared and determined as "immunostaining reagent D".

Meanwhile, thiolated anti-EGFR antibody was also bonded to the nanoparticles having accumulated red fluorescent dye having a maleimide group at the end to prepare "immunostaining reagent E."

Furthermore, the "(1.3) MODIFICATION OF SECONDARY ANTIBODY" was changed to modification by biotin as follows.

(PREPARATION OF BIOTIN-MODIFIED ANTI RABBIT IgG ANTIBODY)

50 $\mu$g of anti-rabbit IgG antibody as a secondary antibody was dissolved in a 50 mM Tris solution. DTT (dithiothreitol) solution was added to the solution such that the final concentration was 3 mM and reacted for three hours at 37°C. After the reaction, the solution was passed through a desalting column "Zeba Desalt Spin Columns" (from Thermo Fisher Scientific Inc., Cat.#89882) to purify a secondary antibody reduced by DTT. 200 $\mu$l of the total amount of the purified antibody was dissolved in a 50 mM Tris solution to prepare an antibody solution. Meanwhile, a linker reagent "Maleimide-PEG2-Biotin" (from Thermo Fisher Scientific Inc., Cat.#21901) was adjusted to be the concentration of 0.4 mM using DMSO. 8.5 $\mu$l of the linker solution was added to the antibody solution and reacted for 30 minutes at 37°C so that biotin was bonded to the anti-rabbit IgG antibody via PEG chain. The reaction solution was passed through the desalting column for purification. Absorbance at the wavelength of 300 nm was measured from the desalted reaction solution using a spectrophotometer ("F-7000" made by Hitachi, Ltd) to calculate the concentration of protein (biotin-modified secondary antibody) in the reaction solution. The concentration of the biotin-modified secondary antibody was adjusted to 250 $\mu$g/ml by a 50 mM Tris solution to prepare a solution of biotin-modified secondary antibody. Staining by the "immunostaining reagent D" was performed as follows. The primary reaction was performed using 0.005 nM of anti-PD-LI rabbit monoclonal antibody (clone "SP142" made by Spring Bioscience (SBS) Corporation) and the secondary reaction was performed using 6 $\mu$g/ml of the solution of biotin-modified anti-rabbit IgG antibody in PBS.

As a result, the number of fluorescent bright points according to the first method was PD-L1:EGFR = 200:1400 and the number of fluorescent bright points according to the second method was PD-L1:EGFR = 100:1400. The cause of the difference is not clear, but the condition of the prepared slide of a patient-derived mouse specimen may be somewhat different from the condition of the specimen slide of an original patient. It is considered that the technique of the present invention can be an effective method in the future for checking the

**Industrial Applicability**

As described above, the present invention is suitable for providing an analysis method and an analysis system of a target biological substance which enable accurate analysis of the number of fluorescent bright points for each immunostaining reagent even in multiple-immunostaining using multiple kinds of immunostaining reagents.

**Description of Reference Numerals**

| 1 | analysis system of target biological substance |
|---|---|
| 10 | fluorescence microscope |
| 12 | stage |
| 14 | objective lens |
| 16 | lens-barrel |
| 18 | ocular lens |
| 20 | imaging element |
| 30 | tissue specimen |
| 40 | lamp |
| 50 | filter set |
| 52 | excitation filter |
| 52a | wavelength width |
| 54 | beam splitter |
| 56 | fluorescence filter |
| 56a | wavelength width |
| 56b | overlapping wavelength width |
| 60 | controller |
| 62 | control unit |
| 64 | storage |
| 70 | display |

**Claims**

1.  An analysis method of a target biological substance for analyzing a tissue specimen with a fluorescence microscope, the tissue specimen being stained with immunostaining reagents including different kinds of fluorescent nanoparticles, the analysis method comprising:

    calculating a luminance ratio of each of the immunostaining reagents for each of filter sets; and
    measuring number of fluorescent bright points in the tissue specimen for each of the filter sets for calculating number of fluorescent bright points for each of the immunostaining reagents based on the luminance ratio.

2.  The analysis method of a target biological substance according to claim 1, wherein, in calculating the number of fluorescent bright points for each of the immunostaining reagents, number of fluorescent bright points L(m) for an immunostaining reagent(m) is calculated from a solution of following simultaneous equations.

$$TB(1) = L(1) + R(1,2) \times L(2) + R(1,3) \times L(3) + \cdots$$

$$TB(2) = R(2,1) \times L(1) + L(2) + R(2,3) \times L(3) + \cdots$$

$$TB(3) = R(3,1) \times L(1) + R(3,2) \times L(2) + L(3) + \cdots$$

$$\cdots \cdots \cdots \cdots \cdots,$$

wherein R(1 to n, 1 to N) represents luminance ratio for each filter set(1 to N) and for each immunostaining reagent(1 to n), TB(1 to N) represents number of fluorescent bright points in the tissue specimen measured for each filter set(1 to N), L(1 to n) represents number of fluorescent bright point for each immunostaining reagent(1 to n), and n, N, and m respectively represent a positive integer and satisfy the expression of 1≤m≤n.

3. The analysis method of a target biological substance according to claim 2, wherein the luminance ratio R(1 to n, 1 to N) is calculated from following General formula (1).

$$\text{General formula (1): luminance ratio } R(m,k) = FL(m,k)/FL(m,p),$$

wherein in the above General formula (1), FL(m,k) represents a total luminance value of fluorescent bright points observed in an entire image of the immunostaining reagent(m) using a filter set(k), FL(m,k) represents a total luminance value of fluorescent bright points observed in an entire image of the immunostaining reagent(m) using a filter set(p), k represents an integer of 1 to N, p represents an integer of 1 to N, and n, N, and m respectively represent a positive integer and satisfy the expression of 1≤m≤n.

4. The analysis method of a target biological substance according to claim 2, wherein
luminance value of one fluorescent bright point is measured for each filter set(1 to N), and
the luminance ratio R(1 to n, 1 to N) is a luminance ratio R(m, k), which is an inclination a described in following General formula (2) representing an approximate straight line indicating a relation between a luminance value X obtained using a filter set(p) and a luminance value Y obtained using a filter set(k).

$$\text{General formula (2): } Y = aX$$

wherein k represents an integer of 1 to N, p represents an integer of 1 to N, and n, N, and m respectively represent a positive integer and satisfy the expression of 1≤m≤n.

5. The analysis method of a target biological substance according to any one of claims 1 to 4, wherein each of the filter sets includes an excitation filter having a wavelength width of 20 to 30 nm and a fluorescence filter having a wavelength width of 30 to 50 nm.

6. The analysis method of a target biological substance according to claim 5, wherein an overlapping wavelength width of fluorescence filters in the filter sets is 10 nm or less.

7. The analysis method of a target biological substance according to any one of claims 1 to 6, wherein the fluorescent nanoparticles are semiconductor nanoparticles or nanoparticles having accumulated fluorescent substance.

8. The analysis method of a target biological substance according to claim 7, wherein the fluorescent nanoparticles have an emission wavelength of 400 to 700 nm.

9. The analysis method of a target biological substance according to claim 8, wherein overlapping emission wavelength of the different kinds of fluorescent nanoparticles is 30% or less, wherein, within a wavelength width of a fluorescence filter in the filter sets corresponding to one kind of the fluorescent nanoparticles, an integrated intensity value of another kind of the fluorescent nanoparticles relative to an integrated intensity value of the one kind of the fluorescent nanoparticles is 30% or less.

10. The analysis method of a target biological substance according to any one of claims 1 to 9, wherein the tissue specimen is collected from an animal model and processed to be a specimen.

11. An analysis system of a target biological substance for analyzing a tissue specimen with a fluorescence microscope, the tissue specimen being stained with immunostaining reagents including different kinds of fluorescent nanoparticles, the system comprising:

a fluorescence microscope to image the tissue specimen stained with the immunostaining reagents,
a controller controlling the fluorescence microscope and comprising:

a storage to store luminance ratio of each of the immunostaining reagents for each of filter sets; and
a calculator to measure number of fluorescent bright points in the tissue specimen for each of the filter sets
and to calculate number of fluorescent bright points for each of the immunostaining reagents based on the
luminance ratio.

*FIG.1*

IMAGING-ELEMENT
SIDE

*1*

*56*

*52*

*50*

*40*

*54*

OBJECTIVE-LENS
SIDE

*16*

*20*

*18*

*60*

*62*

*70*

CONTROL
UNIT

DISPLAY

*10*

*14*

*12*

*30*

*64*

STORAGE

*FIG.2*

PROCESS OF CALCULATING NUMBER OF
FLUORESCENT BRIGHT POINTS

CALCULATE LUMINANCE RATIO — *S1*

CALCULATE NUMBER OF FLUORESCENT BRIGHT POINTS — *S2*

END

# FIG.3

| Immunostaining reagent | Luminance ratio | | |
|---|---|---|---|
| | Filter set for green fluorescence | Filter set for red fluorescence | Filter set for near-infrared ray |
| Immunostaining reagent A (Green) | 1 | 0.08 | 0.01 |
| Immunostaining reagent B (Red) | 0.12 | 1 | 0.08 |
| Immunostaining reagent C (Near-infrared ray) | 0 | 0.1 | 1 |

# FIG.4

APPROXIMATE CURVE
$y = ax + b$

LUMINANCE WHEN USING FILTER SET FOR GREEN

LUMINANCE WHEN USING FILTER SET FOR RED

# FIG.5

| Source of fluorescent bright point | Number of fluorescent bright points | | |
|---|---|---|---|
| | Filter set for green fluorescence | Filter set for red fluorescence | Filter set for near-infrared ray |
| Tissue specimen | 572 | 680 | 453 |
| Immunostaining reagent A (Green) | L(1)=500 | 0.08L(1)=40 | 0.01L(1)=5 |
| Immunostaining reagent B (Red) | 0.12L(2)=72 | L(2)=600 | 0.08L(2)=48 |
| Immunostaining reagent C (Near-infrared ray) | – | 0.1L(3)=40 | L(3)=400 |

# FIG.6

INTENSITY

52a          56a

WAVELENGTH

## FIG.7

INTENSITY

56b    WAVELENGTH

## FIG.8

56a

INTENSITY

—— FLUORESCENT NANOPARTICLE A

--- FLUORESCENT NANOPARTICLE B

30% or less

400    500    600    700    800

WAVELENGTH[nm]

## INTERNATIONAL SEARCH REPORT

| International application No. |
|---|
| PCT/JP2016/071037 |

| A. CLASSIFICATION OF SUBJECT MATTER |
|---|
| *G01N21/64*(2006.01)i, *G01N33/48*(2006.01)i, *G01N33/53*(2006.01)i, *G01N33/533*(2006.01)i, *G02B21/00*(2006.01)i |

According to International Patent Classification (IPC) or to both national classification and IPC

| B. FIELDS SEARCHED |
|---|

Minimum documentation searched (classification system followed by classification symbols)
G01N21/62-21/74, G01N33/48-33/98, G02B21/00

Documentation searched other than minimum documentation to the extent that such documents are included in the fields searched

Jitsuyo Shinan Koho      1922–1996   Jitsuyo Shinan Toroku Koho   1996–2016
Kokai Jitsuyo Shinan Koho   1971–2016   Toroku Jitsuyo Shinan Koho   1994–2016

Electronic data base consulted during the international search (name of data base and, where practicable, search terms used)
JSTPlus/JMEDPlus/JST7580(JDreamIII)

| C. DOCUMENTS CONSIDERED TO BE RELEVANT |
|---|

| Category* | Citation of document, with indication, where appropriate, of the relevant passages | Relevant to claim No. |
|---|---|---|
| Y | WO 2012/029342 A1 (Konica Minolta, Inc.), 08 March 2012 (08.03.2012), paragraphs [0016] to [0033], [0048] to [0061] & US 2013/0157895 A1 paragraphs [0027] to [0053], [0094] to [0114] & EP 2613145 A1 | 1–11 |
| Y | JP 2005-037299 A (Hamamatsu Photonics Kabushiki Kaisha), 10 February 2005 (10.02.2005), paragraphs [0005] to [0006], [0012] to [0013], [0041] (Family: none) | 1–11 |

☒   Further documents are listed in the continuation of Box C.       ☐   See patent family annex.

| | |
|---|---|
| *    Special categories of cited documents: <br> "A"   document defining the general state of the art which is not considered    to be of particular relevance <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
|---|---|
| 13 September 2016 (13.09.16) | 27 September 2016 (27.09.16) |

| Name and mailing address of the ISA/ | Authorized officer |
|---|---|
| Japan Patent Office <br> 3-4-3,Kasumigaseki,Chiyoda-ku, <br> Tokyo 100-8915,Japan | |
| | Telephone No. |

Form PCT/ISA/210 (second sheet) (January 2015)

25

**INTERNATIONAL SEARCH REPORT**

| International application No. |
| --- |
| PCT/JP2016/071037 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | US 2009/0245611 A1 (General Electric Co.), 01 October 2009 (01.10.2009), entire text; all drawings (Family: none) | 1-11 |
| A | JP 8-320292 A (Olympus Optical Co., Ltd.), 03 December 1996 (03.12.1996), entire text; all drawings (Family: none) | 1-11 |
| A | JP 2012-052985 A (Sony Corp.), 15 March 2012 (15.03.2012), entire text; all drawings & US 2012/0056103 A1 & EP 2426481 A1 & CN 102435313 A | 1-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012029342 A